# EUROPEAN PATENT SPECIFICATION

(11) **EP 2 324 051 B1**
(45) Date of publication and mention of the grant of the patent: **23.11.2016**
(21) Application number: 09785078.8
(22) Date of filing: 09.09.2009
(51) Int. Cl.: C07K 14/255, A61K 39/02

(54) **NON-TYPHOIDAL SALMONELLA VACCINES**
IMPFSTOFFE GEGEN NICHT-TYPHOIDE SALMONELLEN
VACCINS CONTRE LES SALMONELLOSES NON TYPHOÏDIQUES

(30) Priority: 09.09.2008 GB 0816462
(43) Date of publication of application: 25.05.2011
(73) Proprietor: THE UNIVERSITY OF BIRMINGHAM, Birmingham B15 2TT (GB)
(72) Inventor: CUNNINGHAM, Adam, West Midlands B30 2HN (GB)
(74) Representative: Ward, David Ian
(86) International application number: PCT/GB2009/002159
(87) International publication number: WO 2010/029293

(56) References cited:
- MATSUI K ET AL: "PROTECTIVE IMMUNITIES INDUCED BY PORINS FROM MUTANT STRAINS OF SALMONELLA-TYPHIMURIUM" MICROBIOLOGY AND IMMUNOLOGY, vol. 34, no. 11, 1990, pages 917-928, XP009128298 ISSN: 0385-5600
- NEGM ROBERT S ET AL: "Macrophages recognize and adhere to an OmpD-like protein of Salmonella typhimurium" FEMS IMMUNOLOGY AND MEDICAL MICROBIOLOGY, vol. 20, no. 3, March 1998 (1998-03), pages 191-199, XP002564407 ISSN: 0928-8244
- SINGH S P ET AL: "IMMUNOPROTECTION BY MONOCLONAL ANTIBODIES TO THE PORINS AND LIPOPOLYSACCHARIDE OF SALMONELLA TYPHIMURIUM" MICROBIAL PATHOGENESIS, ACADEMIC PRESS LIMITED, NEW YORK, NY, US, vol. 21, no. 4, 1 January 1996 (1996-01-01), pages 249-263, XP001147992 ISSN: 0882-4010
- HARA-KAONGA BOCHIWE ET AL: "OMPD BUT NOT OMPC IS INVOLVED IN ADHERENCE OF SALMONELLA ENTERICA SEROVAR TYPHIMURIUM TO HUMAN CELLS" CANADIAN JOURNAL OF MICROBIOLOGY, NRC RESEARCH PRESS, CA, vol. 50, no. 9, 1 September 2004 (2004-09-01), pages 719-727, XP009080360 ISSN: 0008-4166
- CUNNINGHAM ADAM F ET AL: "Salmonella induces a switched antibody response without germinal centers that impedes the extracellular spread of infection" JOURNAL OF IMMUNOLOGY, vol. 178, no. 10, May 2007 (2007-05), pages 6200-6207, XP002564408 ISSN: 0022-1767
- GIL-CRUZ CRISTINA ET AL: "The porin OmpD from nontyphoidal Salmonella is a key target for a protective B1b cell antibody response" PROCEEDINGS OF THE NATIONAL ACADEMY OF SCIENCES OF THE UNITED STATES OF AMERICA, vol. 106, no. 24, June 2009 (2009-06), pages 9803-9808, XP002564409 ISSN: 0027-8424
- MEYER P N ET AL: "Virulence of a Salmonella typhimurium OmpD mutant", INFECTION AND IMMUNITY, AMERICAN SOCIETY FOR MICROBIOLOGY, US, vol. 66, no. 1, 1 January 1998 (1998-01-01), pages 387-390, XP002455109, ISSN: 0019-9567

## Description

### Field of the invention

The present invention relates to vaccines for non-typhoidal Salmonella (NTS) infections, in particular provision in such vaccines of an immunogen which will generate antibodies targeting the outer membrane porin protein (OmpD). Preferably, for example, this may be OmpD itself or a fragment of OmpD retaining the ability to induce antibodies effective against NTS, especially *Salmonella enterica* serovar Typhimurium - referred to as STm below. Such a polypeptide may be provided in combination with an adjuvant carrier molecule. It may be provided as part of a multi-subunit vaccine with one or more other antigens derived from Salmonella.

### Background to the invention

*Salmonella enterica* serovar Typhi (*S.typhi*), *Salmonella paratyphi* A and non-typhoidal salmonellae (NTS) are major causes of disease. Whilst typhoid remains a worldwide public health problem, in some regions such as sub-Saharan Africa, disease from non-typhoidal serovars of *Salmonella enterica,* primarily Typhimurium but also including Enteritidis, are of particular concern as a leading cause of mortality in infants and adults with HIV. This is in stark contrast to NTS infections in the West, which primarily cause a self-limiting gastroenteritis whose impact is largely merely one of economic importance.

*Salmonella* colonise macrophages and resolution of the intracellular phase of the infection is controlled by effector CD4 T cells interacting with infected phagocytes. However, it is well recognised that death from NTS infection is usually associated with bacteraemia and there is much evidence that bacteraemia occurs in individuals that lack specific antibody to restrict extracellular NTS spread and growth (e.g. MacLennan, J. Clin. Invest. (2008) 118:1553). Thus, it has been reported that NTS infections cause a progressive bacteraemia in HIV⁺ adults and children, killing around 50% of infected individuals. Additionally, in sub-Saharan Africa, NTS infections cause fatal disease in non-HIV⁺ infants, primarily between 6 and 24 months, which matches or exceeds the impact of pneumococcal disease. Again, disease severity in such NTS-infected infants is closely associated with bacteraemia; mortality associated with such childhood bacteraemia is approximately 25% in those who reach clinic. The susceptibility of such infants correlates with the loss of maternal antibody and lack of actively acquired immunity to *Salmonellla.*

While vaccines are in use to prevent typhoid caused by *S*. *typhi,* vaccination against typhoid does not appear to induce cross-protection against STm. In some cases, this is clearly due to the differential presence of antigens (e.g. as with use of Vi antigen), but such lack of cross-protection may also extend to killed bacteria and attenuated vaccines. There are currently no vaccines or preventive measures against NTS. Antibiotics such as fluoroquinolines are widely used to treat *Salmonella* infection, but the emergence of multi-drug resistant (MDR) strains of *Salmonella* is an increasing problem and also their administration may be too late after presentation to be effective. Hence, there is a real need for an effective preventive means of combating NTS infection.

The inventors previously reported that STm induces an atypical antibody response in mice that is characterised by the rapid and massive expansion of extrafollicular plasma cells. The induction of this response is T cell-independent, although switching of the response to IgG2a is dependent on CD4 T cell help. These studies also identified that the early, switched antibody response targets antigens in the outer membrane of STm (Cunningham et al. (2007) J. Immunol. 178, 6200-6207). Others have reported that isolated porin preparations from the typhoidal *Salmonella* strain *Salmonella enterica* serovar Typhi (*S. typhi* or ST) also induce an atypically long-lived antibody response in mice (Secundino et al. (2005) Immunology, 117, 59-70). This work showed that both the outer membrane porins OmpF and OmpC contribute to the antibody response with Omp C being the main protein responsible. However, the outer membrane protein (Omp) composition of the outer membranes of ST and STm are different and such work with ST may not be directly applicable to STm. Indeed, Secundio et al. reported that anti-S. *typhi* porin sera did not cross-react with STm.

In the outer membrane of STm, but not S. *typhi,* there is a third porin, OmpD, in addition to OmpF and OmpC. OmpD is a trimeric porin that shares a degree of homology with OmpF and OmpC, primarily in the barrel structure. Whilst OmpF and OmpC expression is regulated by OmpR and is sensitive to change in osmolarity, OmpD expression is enhanced by anaerobis and suppressed by decreases in pH. Singh et al. previously looked at the ability of monoclonal antibodies to STm porins to provide passive immunoprotection against STm in mice (Microbial Pathogenesis (1996) 21, 249-263). No monoclonal antibody to surface-exposed epitopes of OmpC and OmpD was shown to protect against STm infection. A mixture of such antibodies only was found to extend survival time of mice challenged with purified STm outer membrane. However, this represents a very different antibody repertoire than the antibody response to STm *in vivo* and provides no information on the contribution of any individual STm porin to antibody response to STm infection.

WO 2007/062795A (Stifung Tierarztkiche Hochschule Hannover), Selke et al. ("Immunization of pigs to prevent disease in humans: construction of a protective efficacy of salmonella enterica serovar typhimurium live negative-marker vaccine" Infection Immun: Vol 75 pp 2476-2483, 2007) and Can J Mircobiol ("OmpD but not OmpC is involved in adherence of salmonella enterica serovar typhimurium to human cells " Vol 50, pp 719-727, 2004) provide useful insights into the background of the present invention.

Surprisingly, we have now shown that the natural antibody response to OmpD in STm is sufficient to achieve marked reductions in STm bacterial cell numbers. A highly purified STm Omp preparation containing the porins OmpD, OmpC and OmpF was shown to induce a rapid porin protein-specific antibody response in T cell deficient mice associated with induction of a population of innate B1b cells; immunization with such isolated Omps was shown to produce equivalent protective responses, against STm infection, as killed STm. However, infecting porin-immunized mice with OmpD⁻ STm abrogated protection and antibody from porin-immunized mice had impaired binding to OmpD-deficient bacterial cell wall preparations. Furthermore, combined Omp preparations from *S*. *typhi* were shown not to cross-protect, despite the high homology between the OmpC and OmpF porins of ST and STm. This is consistent with an important role for antibody to OmpD in combating NTS infection. Hence, we have now shown that OmpD is a prime candidate for a sub-unit vaccine to NTS.

### Summary of the invention

In a first aspect, the present invention thus provides an immunogenic polypeptide which comprises or consists of:
(i) a polypeptide which is an OmpD of non-typhoidal Salmonella, or an immunogenic fragment thereof having the ability to generate an antibody response against non-typhoidal Salmonella (NTS); or
(ii) a variant of a polypeptide as defined in (i) which has said ability,
   for use in therapy.

The immunogenic polypeptide may comprise of:
(i) a polypeptide which is an OmpD or an immunogenic fragment thereof having the ability to generate an antibody response against NTS, especially *S*. *typhimurium* (STm); or
(ii) a variant of a polypeptide as defined in (i) which has said ability,
for use in therapy (preventive therapy or treatment), more particularly for use in treating or preventing NTS infection and/or disease, especially STm or S. Enteritidis infection and/or disease. Preferably, said immunogenic polypeptide may be native OmpD of non-typhoidal Salmonella, e.g. OmpD as derivable from STm, or an immunogenic fragment thereof. Suitable variants include both natural variants and engineered analogues which maintain the desired antibody specificity.

There is also now provided use of an immunogenic polypeptide which comprises or consists of:
(i) a polypeptide which is an OmpD or an immunogenic fragment thereof having the ability to generate an antibody response against NTS, especially STm; or
(ii) a variant of a polypeptide as defined in (i) which has said ability,
for use in the manufacture of a vaccine composition for use in treating or preventing infection and / or disease by OmpD-containing Salmonella, especially NTS infection and/ or disease such as STm infection and/or disease. Preferably, the vaccine composition will be effective against both STm and *S*. *Enteritidis.* One or more additional immunogenic polypeptides may be employed together with a polypeptide as defined in (i) or (ii) above, e.g. selected from (i) outer membrane porins of NTS and/or ST and/or *S. paratyphi* or immunogenic fragments thereof.

In another aspect, there is provided a vaccine composition which provides an immunogenic polypeptide which comprises or consists of:
(i) a polypeptide which is an OmpD of non-typhoidal Salmonella, or an immunogenic fragment thereof having the ability to generate an antibody response against non-typhoidal Salmonella (NTS); or
(ii) a variant of a polypeptide as defined in (i) which has said ability,
wherein the vaccine composition further comprises one or more additional immunogenic polypeptides.

In a further aspect, there is provided a vaccine composition which provides an immunogenic polypeptide which comprises or consists of:
(j) a polypeptide which is an OmpD of non-typhoidal Salmonella, or an immunogenic fragment thereof having the ability to generate an antibody response against non-typhoidal Salmonella (NTS); or
(ii) a variant of a polypeptide as defined in (i) which has said ability,
and which includes an adjuvant. The antigenic component of such a vaccine may comprise or consist of such a polypeptide antigen. The adjuvant may be separate from this antigen or provided in a fusion protein conjugated to a polypeptide as defined in (i) or (ii) above, e.g. Omp D may be provided conjugated to tetanus toxoid, *Pseudomonas aeruginosa* exotoxin A, cholera toxoid or another adjuvant carrier polypeptide. As indicated above, such a vaccine composition may include other antigens to extend its breadth of range, e.g. to provide a broad range vaccine to both NTS and typhoid-causing Salmonella.

In a further aspect, there is provided a vaccine composition as defined above for use in a method of treating or preventing NTS infection, especially STm infection, and infection by *S*. *paratyphi and*/*or ST.*

In a related aspect, there is provided a method of treating or preventing infection and/or disease by OmpD-containing Salmonella, especially NTS infection and/or disease such as STm infection and/or disease, which comprises administering an immunogenic polypeptide which comprises or consists of polypeptide as defined in (i) and (ii) above.

Omp D is also a feature of the outer membrane of *Salmonella paratyphi.* Hence, it is now extrapolated that OmpD of *S*. *paratyphi,* especially, for example, OmpD of *S*. *paratyphi* A, may also find vaccine use in treating or preventing *Salmonella paratyphi* infection and/ or disease.

Thus,in a related aspect there is also now provided an immunogenic polypeptide which comprises or consists of:
(a) a polypeptide which is an OmpD of *S*. *paratyphi* or an immunogenic fragment thereof having the abilty to generate an antibody response against *S*. *paratyphi*; or
(b) a variant of a polypeptide as defined in (i) which has said ability,
for use in therapy (preventive therapy or treatment). Such an immunogenic polypeptide may be included in a multi-subunit vaccine as discussed above.

From a still further related perspective, there is provided use of an immunogenic polypeptide which comprises or consists of
(a) a polypeptide which is an OmpD of S. *paratyphi* or an immunogenic fragment thereof having the abilty to generate an antibody response against *S*. *parartyphi*; or
(b) a variant of a polypeptide as defined in (i) which has said ability,
for use in the manufacture of a vaccine composition for use in treating or preventing infection and/or disease by OmpD-containing Salmonella, especially *S*. *paratyphi* infection and/or disease. As in the case of vaccine compositions for NTS discussed above, one or more additional immunogenic polypeptides may be employed, e.g. selected from outer membrane porins of NTS (e.g. preferably a NTS OmpD such as the OmpD of STm), and/or ST and /or *S*. *paratyphi* or immunogenic fragments thereof.

In a related aspect, there is provided a vaccine composition which provides an immunogenic polypeptide which comprises or consists of a polypeptide as defined in (a) or (b) above and which includes an adjuvant. The adjuvant may be an adjuvant carrier molecule conjugated to a polypeptide as defined in (a) or (b). This may be a fusion protein, such as any of those described herein.

In a still further related aspect, there is provided a method of treating or preventing infection and/or disease by OmpD-containing Salmonella, especially *S paratyphi,* which comprises administering an immunogenic polypeptide which comprises or consists of polypeptide as defined in (a) or (b) above.

The present invention is also useful in treatment and particularly prophylaxis of bacteraemia. In particular, bacteraemia can be ablated in patients vaccinated by the present immunogen.

Antibodies raised by the present immunogen are preferably IgG or IgM.

The amino acid sequence of OmpD is well known. Reference herein to the OmpD polypeptide may also cover a polynucleotide sequence encoding it.

### Detailed description

OmpD for vaccine use in accordance with the invention may be isolated from bacterial cells or produced recombinantly. As indicated above, truncated versions of OmpD may also be employed provided they retain the required immunogenicity. Analogues of such an OmpD fragment or whole OmpD which retain the ability to raise an antibody response as desired, especially, for example to STm, may be constructed by known techniques of protein engineering. They may have one or more substitutions and/or deletions and/or additions, e.g. one or more conservative substitutions which result in desired immunogencity remaining observable in challenge studies, e.g. in mice challenged with STm.

Variant, fragments or analogues of OmpD are envisaged, provided that they elicit the immune response described herein.

Variants preferably have at least 80%, more preferably at least 85%, more preferably at least 90%, more preferably at least 95%, more preferably at least 99%, more preferably at least 99.5% and most preferably at least 99.9% sequence homology (as appropriate) to the wildtype OmpD amino acid sequence, which is highly conserved between genera, or a polynucleotide sequence encoding it or a complement thereof. Such a determination may be made using the BLAST program, for instance.

OmpD or a fragment thereof or analogue as defined above may be provided in the form of a fusion protein. As indicated above, it may be found convenient or preferable to provide, by way of such a fusion protein, an adjuvant component. Suitable polypeptides to be used for this purpose may be selected from carrier polypeptides well known in the vaccine art for this purpose, some of which are listed above. Flagellins and other polypeptide carriers may also be considered for this purpose. However, equally an adjuvant may be provided separately.

As also indicated above, use of a native OmpD, a fragment thereof or a variant of such a polypeptide which retains the ability to generate desired antibody may be desirably combined with use of one of more further immunogenic polypeptides in a multi-subunit vaccine. Thus, one or more additional immunogenic polypeptides may be provided selected from polypeptides comprising or consisting of (i) an outer membrane porin derivable from NTS, ST or *S*. *paratyphi,* e.g. OmpC of ST or an immunogenic fragment thereof, and (ii) modified versions of such polypeptides which maintain the ability to generate an antibody response to *Salmonella.* Such a polypeptide may be, for example, a native OmpD variant compared to the OmpD of STm or an immunogenic fragment thereof. As noted above, such one or more additional immunogenic polypeptides may be provided to extend specificity, including provision of a broad range vaccine effective against NTS, especially Stm, and additionally against typhoidal salmonella such as S. *typhi* and/or *S.paratyphi.*

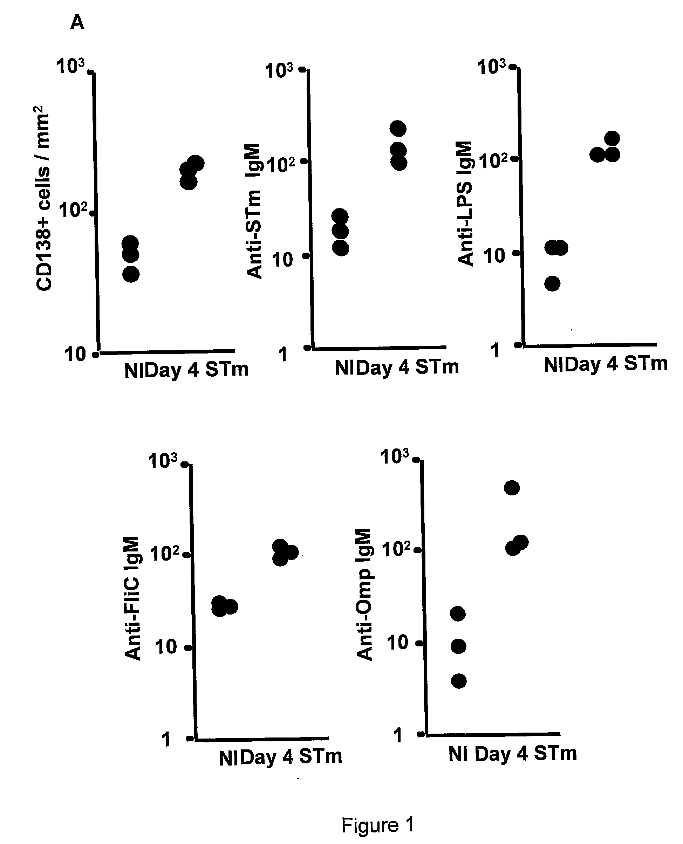

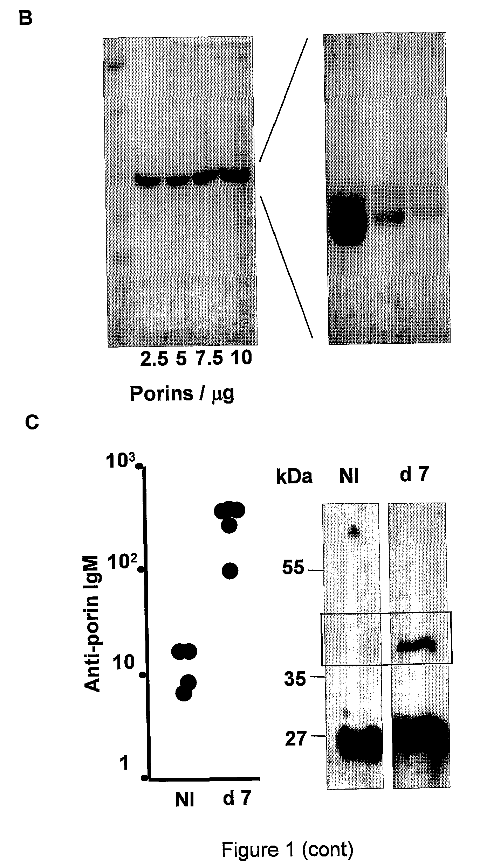

In addition to the studies shown in Figures 1-4, we have conducted further experiments and have come to a number of additional findings, as set out below.

We have determined, by structural assays, that the isolated porins used in these studies maintain their native properties, showing that the antibodies target the functional porin protein.

The present immunogens have now also been shown to induce porin-specific plasma cells in the spleen of mice 4 days after infection. This shows that response are rapidly induced to these immunogens, which is clearly beneficial to a vaccine.

We have found that T cells are needed for the effective production of switched IgG antibody to porins. However, T cells are not necessary for the induction of IgM. Thus, the antibodies raised against the immunogen are preferably IgM or IgG. In the case of IgG, it is preferred that the vaccinee or patient has a sufficient T-cell count to facilitate the induction of IgG. If they do not, then other immunoglobulin types will suffice, such as IgM.

Porin immunization has been shown to be sufficient to ablate bacteraemia, a key marker of disease severity. Thus, the prophylaxis of bacteraemia is particularly preferred, although treatment is also envisaged.

In fact, we have also shown that immunizing a patient or vaccinee twice confers a greater benefit than a single immunization. It is therefore preferred that the treatment comprises at least two or more, and preferably 3 or more or, 4 or more, vaccinations comprising the present immunogen. Two vaccinations are particularly preferred however. These may be suitably spaced apart over two or more days.

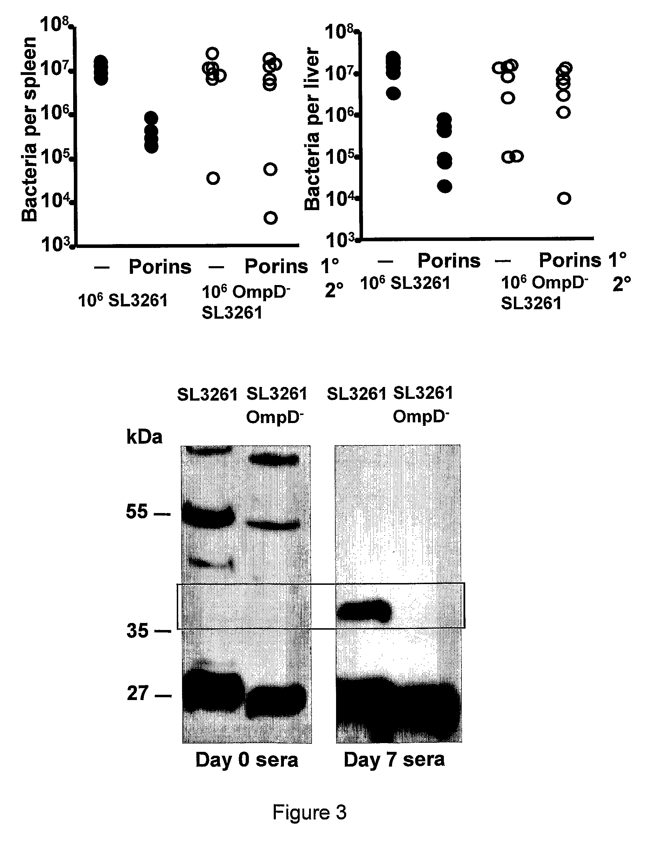

We have confirmed that infection of mice, with bacteria lacking OmpF and OmpC (the other porins found in the purified porin preparation) but that still express OmpD, is impaired and bacteraemia ablated after immunization with the present immunogen. In contrast, infection of porin-immunized mice with bacteria that lack OmpD but that are OmpF and OmpC sufficient is not impaired (see Figure 3), with bacteraemia moderated to a more marginal level. In addition, antibody binding to porins in the cell wall is still retained.

Lastly, we have shown that the murine model also respond to vaccines such as the Typhim polysaccharide (a current vaccine against typhoid). This may be important because children under 2-3 do not respond to this vaccine and it is children of this age who are susceptible to NTS. This supports the importance of these B1 b cells. Preferably, the Typhim polysaccharide can also be used as an adjuvant in the present invention, in particular a vaccine for children of 3 years of age or under.

Where reference is made to a particular use, it will be appreciated that this encompasses the corresponding method of treatment. In other words, the invention also provides for method of treatment and methods of vaccination of an individual or a population, such methods for instance comprising administering the present immunogen, optionally with an adjuvant to a patient in need thereof or to an individual or a population to be vaccinated.

Means suitable for delivery of the immunogen, are well known in the art, but may include use of a soluble immunogen, encapsulation within liposomes, or formation of an ISCOM (Immune Stimulating Complex). Delivery may also be via a polynucleotide encoding the immunogen, for instance delivered via a plasmid or viral (such as a retroviral) vector comprising said polynucleotide. Thus, the immunogenic polypeptide may, preferably, be expressed from a polynucleotide. Use of alum-based and other adjuvants is also preferred.

The invention is further described below in the following exemplification with reference to the following figures:

### Brief description of the figures

Figure 1. Results of studies showing that porins induce a rapid, specific antibody response in the absence of T cells. (A) By 4 days after ip infection with Stm T cell-deficient mice can induce a rapid, extrafollicular, splenic plasma cell response to STm that results in marked increases in serum antibody to LPS and Omp, but not flagellin. (B) Highly purified Omps from STm- OmpC, OmpD and some OmpF (C) Porins rapidly induce T-independent IgM antibody that is specific to the porin proteins. All mice were immunized / infected ip. NI=non-immunized.

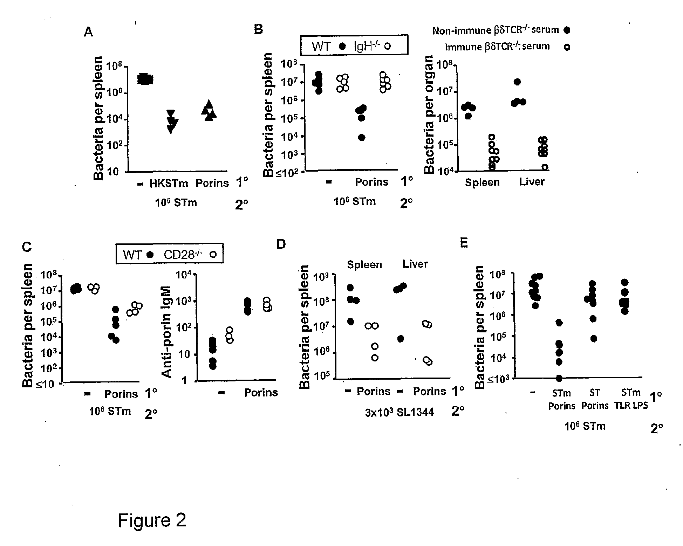

Figure 2. Results showing that antibody against STm porins can protect against STm as further discussed below. (A) Mice that were immunized with heat-killed STm or purified porins (Fig. 1B) showed an approximately equivalent 100-1000 fold drop compared to non-immunized mice in the numbers of bacteria in the spleen 4-5 days after infection with STm. (B) The protection conferred by immunization with porins was mediated by B cells since infecting porin-immunized mice that lacked B cells (IgH-/- mice) conferred no advantage (left panel). Furthermore, opsonising bacteria with antibody from porin-immunized T cell-deficient mice prior to infection conferred significantly more protection than opsonising with sera from non-immunized mice (right panel). (C) In CD28-deficient mice that cannot effectively switch to IgG1 or IgG2a but still have B cells and IgM then antibody to porins is still induced and confers protection but this protection is not as great as when switched antibody is induced. (D) Immunization with porins can impair infection with a strain of STm (SL1344) highly virulent in susceptible strains of mice. (E) Whilst STm porins containing OmpD, OmpC and OmpF could confer benefits against infection, ST porins prepared in a similar manner and containing OmpF and OmpC could not. Furthermore, this benefit was not due to LPS since immunization with a highly pure commercial preparation of STm LPS (Axxora) also failed to confer protection against infection.
Figure 3. Results showing that porin protection can be mediated, in large part, through OmpD. (A) Mice were primed with porins for 35 days before challenge with STm or OmpD-deficient Stm and responses assessed 5 days later. Bacterial numbers in the spleen and liver are similar in the OmpD-defcieint STm group, irrespective of whether mice were immunized with porins beforehand. (B) The reactivity of IgM in sera from naive T cell-deficient mice or T cell-deficient mice immunized for 7 days with porins. The sera were tested against two sets of antigens: cell wall fractions from STm or STm lacking OmpD. The binding of immune sera to the porin fraction (boxed) from OmpD-deficient STm is shown to be diminished.

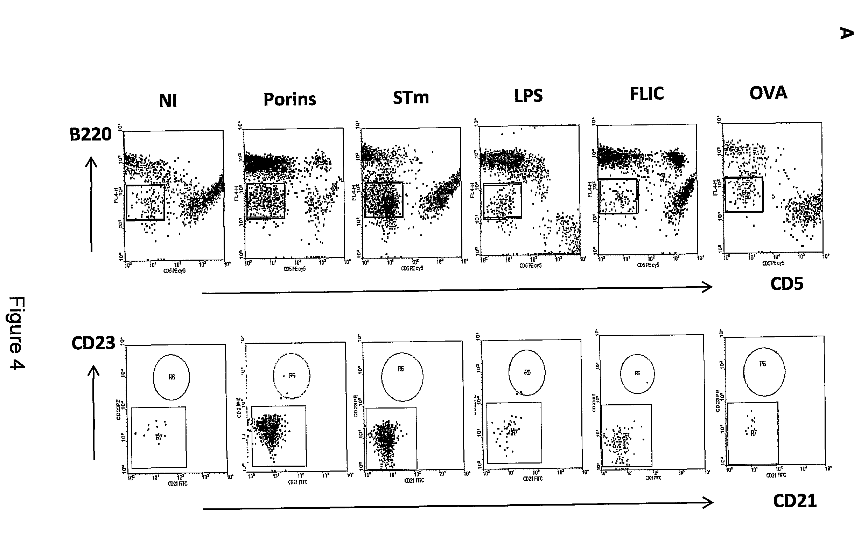

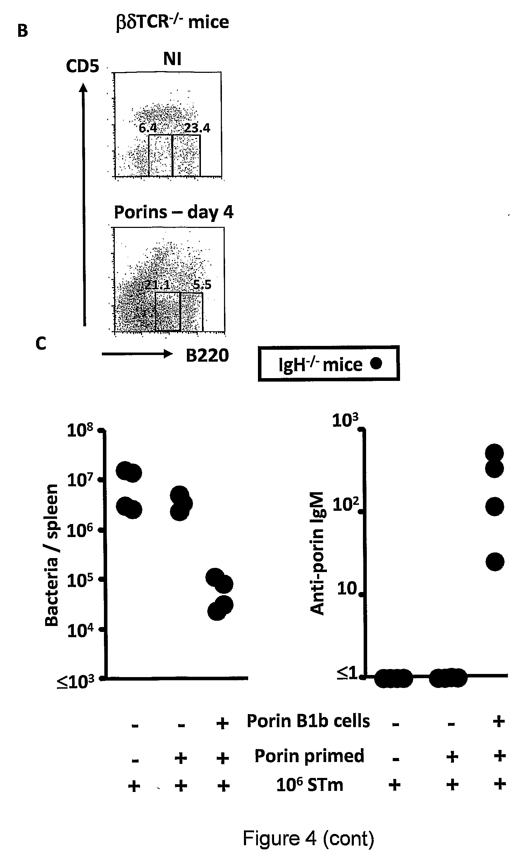

Figure 4. Stm and purified porins induce a T-independent B1b cell population that can protect against infection. (A) Peritoneal cavity lymphocytes assessed before, or 4 days after, antigen. The top row shows B220 and CD5 expression. Below is the CD21 and CD23 expression for the B1b, B220^{int}CD5- population. (B) B1b cells in non-immunized and porin-immunized T cell deficient mice. NI = non-immunized (C) B1 b cells transferred into IgH-/- (B cell-deficient) mice and immunized with porins confer protection against STm. Chimeras and B cell deficient mice were primed with porins for 21 days before infection for 5 days. Chimeras were protected against infection (left panel) and produced anti-porin IgM. B cell deficient animals without B1 b cells but primed with porins were not protected. Characterisation of these cells has been improved upon in our recent publication: Cunningham et al. (PNAS, June 16, 2009, vol 106, no. 24, pp 9803-9808).

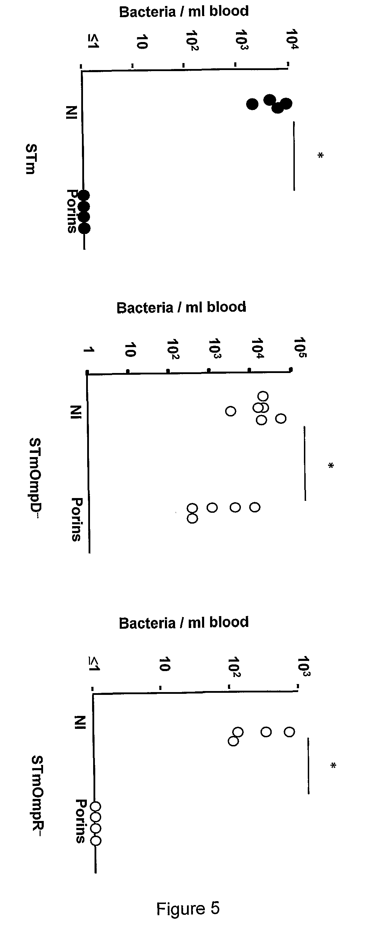

Figure 5. Immunization with porins impairs bacteraemia after STm infection. Blood bacterial counts from NI and porin-immunized WT mice infected with 5x106 STm (left panel) or STm lacking OmpD (centre panel) or STm lacking OmpR (right panel) for 5 days. NI - non-immunized. * - signifies P < 0.05

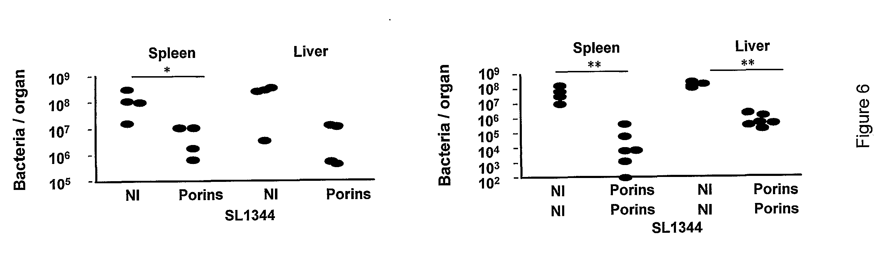

Figure 6. Multiple immunizations with porins confer greater protection to STm infection than single infection. Spleen and liver bacterial counts from WT mice that were immunized once (left panel) or twice (right panel) with porins and infected with 3x103 STm strain SL1344 for 4 days. NI - non-immunized. * - signifies P < 0.05; ** - signifies P < 0.01

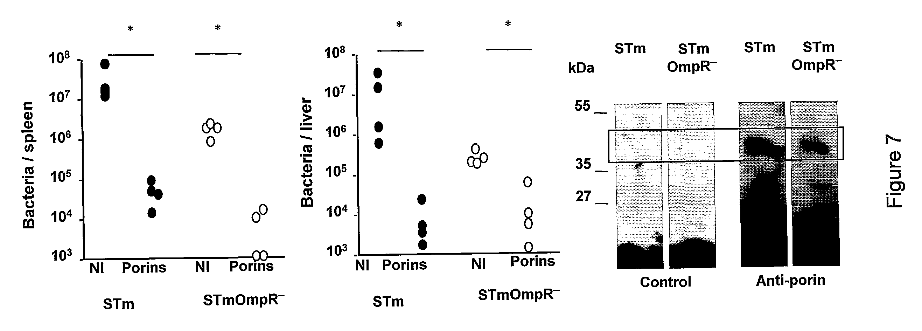

Figure 7. Infection with OmpR-deficient STm, that lack OmpF and OmpC but retain OmpD expression, is moderated after porin immunization. Non-immunized (NI) or porin-immunized WT mice were infected with 5x106 STm (closed circles) or 5x106 STm lacking OmpR (STmOmpR- strain RAK83; open circles) for 5 days and splenic (left panel) and liver (centre panel) bacterial numbers enumerated. Immunoblot analysis (right) of IgM from NI or porin-immunized TCRbd-/- mice against cell walls isolated from STm or STm lacking OmpR. The boxed region shows the approximate Mr of the porin OmpD. The Mann-Whitney test was applied to compare the statistical significance between groups linked by bars. * - signifies P < 0.05.

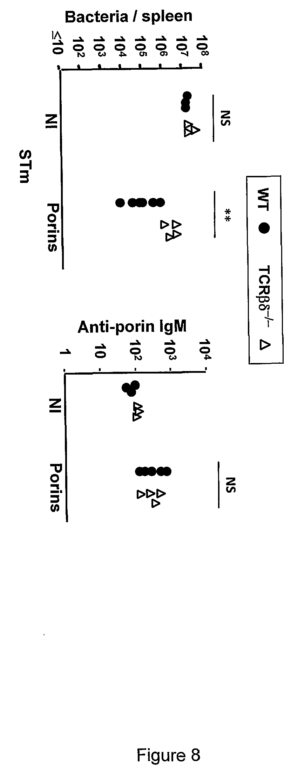

Figure 8. The induction of IgG enhances the benefits of porins immunization. Induce B1 b cells. NI or porin-immunized WT or TCRbd-/- mice were infected with 5x106 STm for 5 days and splenic bacterial numbers enumerated (left panel). Right panel shows serum IgM titers from 5 day STm infected NI and porin-immunized WT and TCRbd-/- mice. NI - non-immunized. ** - signifies P < 0.01; NS - not significant.

The present invention will now be described in the following non-limiting Examples.

### Example 1

### Materials and Methods

### Mice, bacterial strains and immunogens.

Wild-type (WT) mice were obtained from in house colonies. Sources of gene-deficient mice have been reported elsewhere (Cunningham et al. (2007) J. Immunol. 178, 6200-7 and Gaspal et al. (2008) J. Immunol. 180, 2824-9) with the exception of βδTCR-deficient mice, which were obtained from Jax. All mice and groups were age (6-12 weeks) and sex-matched before use. *Salmonella enterica* serovar Typhimurium SL1344 is a WT strain and SL3261 is a well-described AroA-deficient attenuated strain. OmpD-deficient STm were generated on a SL3261 background.

Total Omp preparations were generated as described previously by 2% (v/v) Triton X-100 extraction from cell envelopes and harvesting by centrifugation. Purified porins were generated using well-established methods (Salazar-Gonzalez et al. (2004) Immunol. Letts 93, 115-22) from ST (strain 9933) and STm (strain ATCC 14028).

In brief, porins were isolated from the supernatant of nuclease and MgCl₂ treated sonicated cells followed by repeated treatment with buffers containing Tris HCl with SDS and a final purification by FPLC on a Sephacryl S-200 column. Porin containing fractions were assessed by gel electrophoresis before extensive dialysis against PBS containing 0.1% SDS After purification, porins were stored at RT or - 80°C. Limulus amebocyte lysate assay showed LPS contamination to be 0.06 EU / 480 µg porins. Protein identity was confirmed by trypsin digest and analysis using a Quadrupole Time of Flight Mass Spectrometer at The Functonal Genomics and Proteomics Unit at The University of Birmingham.

TLR grade STm LPS was purchased from (Axxora; product ALX-581-011-L002) and was highly purified to eliminate any cross-reactivity with TLR2. Flagellin was generated as described elsewhere and purified to homogeneity by immunoprecipitation. OVA, Imject grade, was purchased from Thermo Scientific.

### Immunization and infection of mice and opsonisation experiments

Mice were immunized i.p. with proteins or LPS at 20 µg in PBS per animal, unless otherwise stated. Mice were infected with attenuated strains of bacteria at 10⁵ or 10⁶ organisms per animal as described for each experiment. Mice were infected i.p. with 3 x10³ WT SL1344 bacteria. At the specified times after infection, mice were sacrificed and tissues isolated. The number of bacteria per spleen or liver was identified by direct culturing of tissues as described previously. Experiments involving infection of mice after opsonisation of bacteria *in vitro* were performed as described previously. Before opsonisation, sera were complement-inactivated by heating to 56°C for 20 minutes before dilution and mixing with bacteria for 30 minutes at room temperature. In each experiment, a single serum was used per mouse and usually multiple mice per serum sample were used. For each experiment bacterial viability and failure to agglutinate were assessed.

### Immunohistology, ELISA, Gel electrophoresis and Western blotting

Immunohistology for the detection of plasma cells (CD138+ cells, antibody from BD Pharmingen) and IgD+ cells was described as previously (Cunningham et al. (2007) J. Immunol, 178, 6200-7) with the exception that the anti-IgD used was a sheep polyclonal (Abcam ab9177 - 1;1:1000) and the streptavidin - APC complex used was the Vectastain ABC-AP kit from Vector Laboratories. Sera were assessed for antibody levels by ELISA using methods previously described, where antigens were coated at 5 µg/ml for protein antigens and LPS or 10 µg/ml for whole organism. Goat anti-mouse IgM antibodies linked to alkaline phosphatase (Southern Biotechnology Associates) were used and colour was developed using Sigma Fast p-nitrophenyl phosphate tablets. Proteins were separated on 4 - 20% gradient gels (Thermo Scientific) or on 12% or 15% gels using standard methods. For blotting, after electrophoresis proteins were transferred onto PVDF membrane and the membrane blocked. The membrane was blocked overnight and washed before endogenous HRP was eliminated using SG substrate (Vector Laboratories). After washing the membrane was incubated with the appropriate sera overnight and then probed with HRP-conjugated, goat-anti-mouse IgM (Southern Biotech) for 2 hours at room temperature. The membrane was then washed before development using Supersignal Chemiluminescense (Thermo Scientific).

### FACS analysis and cell sorting

Peritoneal exudate cells (PEC) were obtained by lavage and stained with one or more of these anti-mouse mAb-IgM FITC, CD3 PerCPcy5, CD5 PEcy5, B220 APC, CD21FITC and CD23PE (all E- bioscience; clones eB121-15F9, 145-2C11, 53-73, RA3-6B2, 8D9, B3B4 respectively). Cells were analyzed with a FACSCallibur flow cytometer (BD Biosciences) and data analyzed using WinMDI 2.9. For transfer, cells were isolated from mice immunized 4 days previously with porins and B1b cells (B220^{int} CD5) and sorted using a MoFlo cell sorter. Cells (2 x 10⁵) were transferred into IgH -/- mice and 3 days later were immunized i.p. with 20 µg of porins. Success of transfer was confirmed by anti-IgM and anti-porin serum antibody at 5 days later and before challenge with STm.

### Statistics

Statistics were calculated using the nonparametric Mann-Whitney sum of ranks test. The p values were calculated using the Analyze-It program.

### Results

### I. Stm and a STm total outer membrane protein preparation induce a rapid T-independent plasma cell response.

As indicated above, it had previously been established that STm could induce a rapid and extensive B cell response and that a major target of the early, switched response was the outer membrane proteins, but not LPS or FliC. To dissect this response in greater detail, mice were used that completely lacked T cells (βTCR-deficient mice). Infection with attenuated *Salmonella* resulted in a large increase in CD138⁺ plasma cells by day 4 of infection and this was paralleled by a rapid induction of serum IgM against *Salmonella* (Fig. 1A). More detailed analysis of the antibody response showed that there was a strong induction of antibody to LPS and a total outer membrane protein preparation but a much less marked induction of antibody to FliC (Fig. 1A).

### 2. Immunization of T cell-deficient mice with a highly purified STm porin preparation.

To extend the above-noted studies, the response to a highly purifed outer membrane porin preparation containing OmpC, OmpD and OmpF was investigated in T cell-deficient mice (Fig. 1B). ELISA and Western blot of sera from the immunized mice confirmed induction of antigen-specific IgM (Fig. 1C). Thus antibody to STm porins is induced in the absence of T cell help.

### 3. Porin-induced protection against STm infection is mediated entirely through B cells.

It was then assessed whether the antibody induced after immunization with a single dose of purified porins could impair infection with STm. WT mice were immunized with porins and challenged with attenuated *Salmonella* 35 days later. As an initial comparison, the level of protection offered by porins was compared to that offered by heat-killed bacteria. Immunization with either purified porins or killed bacteria reduced splenic bacterial burdens by a median 2-3 logs (Fig 2A). Indeed, antibody induced to porins by the 4th day of immunization could reduce infection by approximately 50% (data not shown). To assess whether B cells mediated this protection, WT mice and mice lacking all B cells (IgH-deficient mice) were immunized with porins and infected after 35 days. This showed a complete ablation of protection conferred by Omps in the absence of B cells (Fig. 2B). In contrast, opsonisation of bacteria with serum from porin-immunized T cell-deficient mice was sufficient to impair infection.

Since B cells had been shown to be necessary for protection and IgM alone could confer benefit, it was then determined whether there was an additive benefit from switched antibody, over and above IgM, by immunizing CD28-deficient animals with Omps and infecting 35 days later. Whilst the induction of IgM to porins is unimpaired in CD28-deficient mice (Fig.2C) these mice are unable to induce switching. These studies showed that bacterial burdens were approximately 10-fold greater in CD28-deficient animals indicating that 90% of the protection observed was mediated by IgG (Fig 2C). These results were similar to those found in equivalent experiments using CD40L-deficient mice, which also have defective switching.

Next it was assessed whether anti-porin antibody could reduce the bacterial burden in mice infected with the WT STm SL1344. This showed that bacterial burdens in the spleen and liver 4 days after infection had median bacterial numbers 20 and 40 fold lower respectively compared to mice that were non-immunized (Fig. 2D).

Lastly, it was examined whether any residual LPS contamination could account for these findings and whether porins from ST could provide similar benefits. Mice were immunized with commercially available TLR grade LPS from STm or with porins from ST and then infected 35 days later. Surprisingly, TLR grade LPS did not confer protection against infection with attenuated bacteria (Fig. 2E). Similarly, as noted above, ST porins did not confer selective protection. The lack of protection offered by porins from ST was surprising, considering the near complete identical homology between OmF and OmpC from ST and STm.

### 4. Protection conferred by porins to STm is mediated by antibody to OmpD

To test whether OmpD contributed to the protection offered by STm porin immunization, mice were immunized with porins from STm and after 35 days infected with either STm or STm lacking OmpD. This showed that immunization conferred no benefit on mice infected with OmpD-deficient STm (Fig. 3). It was then assessed how antibodies induced to Stm porins in T cell-deficient mice bind to an outer membrane preparation from OmpD-deficient bacteria. Antibody did not bind to the porin fraction of outer membrane protein preparations from bacteria lacking OmpD. This indicates that antibody to OmpD is an important target of antibody to STm and can provide protection against subsequent STm infection.

### 5. Porins and STm, but not LPS nor flagellin, induce a population of peritoneal CD19+B220^{int}CDS - B cells

To assess whether innate B cell populations were induced after immunization with STm and STm porins, WT mice were immunized and peritoneal responses assessed on day 4 after immunization. Porins induced a population of B cells that were characterized by having a B220^{int}CD5⁻ phenotype, characteristics of B1b cells that are CD19⁺ and IgM⁺ but have low expression of CD21 and CD23 (Fig. 4A and data not shown). Whilst this population was also induced by live STm, it was not induced by highly purified TLR grade LPS nor FliC nor OVA in 0.1% SDS (Fig. 4A). To confirm the T-independence of this B cell population, CD28-deficient and βδTCR-deficient mice were immunized with isolated proteins or *Salmonella* (Fig. 4B; data shown for βδTCR-deficient mice but similar results for CD28-deficient mice). The B1b population was found to be induced after immunization with STm and porins in the absence of T cells. Thus, both STm and STm porins, but not LPS, induce a peritoneal population of B cells that have a B1b cell phenotype. It has also been shown that STm lacking OmpR (the regulator of OmpF and OmpC expression and so lacking expression of both these proteins) also induces such B cells (data not shown).

### 6. Antibody from B220⁺CD5⁻ B1b cells is sufficient to provide protection

To determine whether antibody produced by B1 b cells induced in response to STm porins is sufficient to provide protection, chimeric mice were generated. Peritoneal B220⁺CD5⁻ cells were isolated from naive WT mice immunized 4 days earlier with porins and 2 x 10⁵ B220⁺CD5⁻ cells were sorted and transferred into B cell-deficient mice. Forty eight hours later, B1 b cell recipients and B cell-deficient mice were immunized with STm porins. After 21 days, the success of transfer was confirmed by measuring anti-porin IgM from the immunized chimeras and control mice. The next day, immunized and naive mice were infected with 10⁶ attenuated Salmonella ip and bacterial burdens and serum antibody was assessed 5 days later. This showed that only those mice that had anti-porin antibody and B cells at the time of infection were able to impair bacterial infection (Fig. 4C). Mice that were immunized with porins in the absence of B cells had bacterial numbers equivalent to non-immunized mice. Therefore, antibody from the transferred B1 b cells was able to limit protection.

### Summary

To summarise the key points from these studies, it has been shown:
1. The T-independent response to STm is selective with early, pronounced responses to LPS and Omp but not the flagellin molecule FliC (Fig. 1A). A highly purified Omp preparation containing the porins OmpD, OmpC and OmpF induces a rapid porin protein-specific antibody response in T cell-deficient mice (Fig. 1B and 1C);
2. Immunization with porins or killed bacteria conferred similar protection against STm and protection by porins was wholly B cell-dependent. IgG enhanced the effectiveness of IgM approximately 10-fold. Lastly, protection was not induced to pure LPS or flagellin or porins from *S*. *Typhi,* which lack OmpD (see Figure 2 and data not shown);
3. Infecting porin-immunized mice with OmpD-deficient STm abrogated protection (Fig. 3A) and antibody from porin-immunized mice had impaired binding to an outer membrane preparation from OmpD-deficient STm (Fig. 3B);
4. Porins and STm, but not LPS, FliC or OVA, induce peritoneal B220^{int}CD5⁻B1b cells in WT and T cell-deficient mice (Figs. 4A and B). Transfer of B1 b cells into B cell-deficient mice with porin immunization could protect against STm infection and this depended upon antibody (Fig. 4C); and

B1 b cells are self-renewing and interestingly have been identified as being important in other infections such as those caused by *Borrelia hermensii* and S*treptooccus pneumoniae.* Indeed it has been reported that B1b cells can also respond to a bacterial protein of *Borrelia -* the complement factor H-binding protein.

That OmpD protein is the major target of the antibody induced to STm porins and that LPS is not of significance to this response was shown by:
(i) using porins purified such that they had undetectable levels of LPS contamination (< 0.01 EU / 100 µg of porins);
ii) immunizing with highly purified STm LPS, as controls to show that this did not protect;
iii) showing loss of benefit of porin immunization after infection with bacteria deficient for OmpD but sufficient for LPS;
iv) loss of binding of antibody from porin-immunized mice to OmpD-deficient bacterial cell walls;
v) all reductions in bacterial titres were ablated if B cells were not present during immunization;
vi) porins induced a B-1b cell population not induced by LPS that was sufficient to confer protection to infection.

Together these strongly implicate that antibody to porins and in particular OmpD can reduce the level of NTS infection.

### Conclusion

It is thus indicated that antibody response to OmpD can reduce STm infection and that this response is mediated through B cells and at least in part via induction of a T-independent B1 b cell population that is sufficient on its own to impair STm infection.

### Example 2

The following further work was carried out following the protocols in Example 1, where appropriate:
1. In NTS, bacteraemia is frequently reflective of disease severity. Therefore, we studied the effects of porin immunization on bacteraemia induced by subsequent infection with STm or STm lacking OmpR (which results in deficient OmpF and OmpC expression) or OmpD-deficient STm. We showed that porin immunization can ablate bacteraemia to STm infection (Figure 5, left panel) and was seen after infection of porin-primed mice with OmpR-deficient STm (Figure 5, central panel) but much less so after infection with OmpD-deficient STm (Figure 5, right panel). This indicates that antibody to porins, in particular OmpD, can markedly reduce bacteraemia.
2. A single immunization with porins is sufficient to impair the bacterial burden to STm. To assess whether immunizing twice augments the benefit of porin immunization mice were immunized twice with porins and infected with STm strain SL1344. This showed that immunizing twice confers greater benefit than a single immunization (Figure 6).
3. Infection of porin-immunized mice with bacteria that lack OmpD reveals porinimmunization (lacking OmpD) confers no benefit in terms of reducing bacterial numbers in tissues. In contrast, infection of mice with bacteria that lacks OmpF and OmpC, but still expresses OmpD, is impaired after porin immunization. This shows that anti-OmpF or OmpC antibody is not as potent at controlling infection as anti-OmpD antibody. Furthermore, antibody from porin-immunized mice could bind to porins in the cell wall of bacteria lacking OmpF and OmpC, but not OmpD (Figure 7).
4. Since the effects at reducing infection after porin immunization is B cell-mediated and T-independent we assessed whether switched Ab conferred additional benefit over and above IgM. To test this wildtype (WT) and T cell-deficient mice were immunized with porins and infected 35 days later. Whilst porin-specific IgM was induced in all groups after infection (Figure 8), porin-specific IgG was largely undetectable in T cell-deficient mice. Bacterial burdens were approximately 40-fold greater in T cell-deficient mice. Taken together with the finding that porin immunization was only effective in the presence of B cells and all groups of naive animals had similar bacterial numbers 5 days after infection these data suggest that ≥90% of the benefit is IgG mediated (Fig. 2C).

## Claims

1. An immunogenic polypeptide which comprises or consists of:
(i) a polypeptide which is an OmpD of non-typhoidal Salmonella, or an immunogenic fragment thereof having the ability to generate an antibody response against non-typhoidal Salmonella (NTS); or
(ii) a variant of a polypeptide as defined in (i) which has said ability,
for use in therapy.

2. An immunogenic polypeptide for use as claimed in claim 1, wherein the immunogenic polypeptide is for use in a method of treating or preventing NTS infection and/or disease.

3. An immunogenic polypeptide for use as claimed in claim 2, wherein the immunogenic polypeptide is for use in a method of treating or preventing Salmonella Typhimurium (STm) infection and/or disease.

4. An immunogenic polypeptide for use as claimed in any one of claims 1 to 3, wherein the immunogenic polypeptide comprises or consists of OmpD obtainable from STm, or an immunogenic fragment thereof.

5. An immunogenic polypeptide for use as claimed in claim 1, wherein the immunogenic polypeptide is a fusion protein in which a polypeptide sequence as defined in (i) or (ii) is linked to a second polypeptide sequence.

6. An immunogenic polypeptide for use as claimed in claim 5 wherein said second polypeptide sequence is an adjuvant carrier protein.

7. A vaccine composition comprising an immunogenic polypeptide which comprises or consists of:
(i) a polypeptide which is an OmpD of non-typhoidal Salmonella, or an immunogenic fragment thereof having the ability to generate an antibody response against non-typhoidal Salmonella (NTS); or
(ii) a variant of a polypeptide as defined in (i) which has said ability,
wherein the vaccine composition further comprises one or more additional immunogenic polypeptides.

8. A vaccine composition as claimed in claim 7 wherein said one or more additional immunogenic polypeptides are selected from polypeptides comprising or consisting of:
(i) an outer membrane porin of NTS, S. *typhi* (ST) or S. *paratyphi* or an immunogenic fragment thereof; and
(ii) analogues thereof which maintain the ability to generate an antibody response to Salmonella.

9. A vaccine composition comprising an immunogenic polypeptide which comprises or consists of:
(i) a polypeptide which is an OmpD of non-typhoidal Salmonella, or an immunogenic fragment thereof having the ability to generate an antibody response against non-typhoidal Salmonella (NTS); or
(ii) a variant of a polypeptide as defined in (i) which has said ability,
wherein the vaccine composition further includes an adjuvant.

10. A vaccine composition as claimed in claim 9 wherein said adjuvant is an immunogenic polypeptide in the form of a fusion protein in which a polypeptide sequence as defined in (i) or (ii) is linked to a second polypeptide sequence, optionally wherein said second polypeptide sequence is an adjuvant carrier protein..

11. A vaccine composition as claimed in claim 9 or claim 10 which includes one or more additional immunogenic polypeptides.

12. A vaccine composition as claimed in claim 11 wherein said one or more additional polypeptides are selected from polypeptides comprising or consisting of:
(i) an outer membrane porin of NTS, ST or *S*. *paratyphi* or an immunogenic fragment thereof; and
(ii) analogues thereof which maintain the ability to generate an antibody response to Salmonella.

13. A vaccine composition as claimed in claim 9 or claim 10, for use in a method of treating or preventing NTS infection, especially STm infection.

14. A vaccine composition as claimed in claim 11 or claim 12, for use in a method of treating or preventing NTS infection, especially STm infection, and infection by S. *paratyphi and*/*or ST.*

## Patentansprüche

1. Immunogenes Polypeptid, umfassend oder bestehend aus:
(i) ein/einem Polypeptid, das ein OmpD von nicht-typhösen Salmonellen ist, oder ein immunogenes Fragment davon, mit der Fähigkeit, eine Antikörperreaktion gegen nicht-typhöse Salmonellen (NTS) zu erzeugen; oder
(ii) eine/einer Variante/Varianten eines Polypeptids, wie in (i) definiert, die die genannte Fähigkeit aufweist,
für die Verwendung in der Therapie.

2. Immunogenes Polypeptid für die Verwendung nach Anspruch 1, wobei das immunogene Polypeptid für die Verwendung in einem Verfahren für die Behandlung oder Verhinderung einer NTS-Infektion und/oder einer NTS-Erkrankung ist.

3. Immunogenes Polypeptid für die Verwendung nach Anspruch 2, wobei das immunogene Polypeptid für die Verwendung in einem Verfahren für die Behandlung oder Verhinderung einer Salmonella Typhimurium (STm)-Infektion und/oder einer Salmonella Typhimurium (STm)-Erkrankung ist.

4. Immunogenes Polypeptid für die Verwendung nach einem der Ansprüche 1 bis 3, wobei das immunogene Polypeptid OmpD umfasst oder daraus besteht, erhältlich von STm, oder ein immunogenes Fragment davon.

5. Immunogenes Polypeptid für die Verwendung nach Anspruch 1, wobei das immunogene Polypeptid ein Fusionsprotein ist, bei dem eine Polypeptidsequenz, wie in (i) oder (ii) definiert, mit einer zweiten Polypeptidsequenz verbunden ist.

6. Immunogenes Polypeptid für die Verwendung nach Anspruch 5, wobei die genannte zweite Polypeptidsequenz ein Adjuvans-Carrierprotein ist.

7. Impfstoffzusammensetzung, umfassend ein immunogenes Polypeptid, umfassend oder bestehend aus:
(i) ein/einem Polypeptid, das ein OmpD von nicht-typhösen Salmonellen ist, oder ein immunogenes Fragment davon, mit der Fähigkeit, eine Antikörperreaktion gegen nicht-typhöse Salmonellen (NTS) zu erzeugen; oder
(ii) eine/einer Variante/Varianten eines Polypeptids, wie in (i) definiert, die die genannte Fähigkeit aufweist,
wobei die Impfstoffzusammensetzung ferner ein oder mehrere zusätzliche immunogene Polypeptide umfasst.

8. Impfstoffzusammensetzung nach Anspruch 7, wobei die genannten ein oder mehreren zusätzlichen immunogenen Polypeptide aus Polypeptiden ausgewählt sind, umfassend oder bestehend aus:
(i) ein/einem Außenmembranporin von NTS, S. typhi (ST) oder S. paratyphi, oder ein immunogenes Fragment davon; und
(ii) Analoga davon, die die Fähigkeit behalten, eine Antikörperreaktion gegenüber Salmonellen zu erzeugen.

9. Impfstoffzusammensetzung, umfassend ein immunogenes Polypeptid, umfassend oder bestehend aus:
(i) ein/einem Polypeptid, das ein OmpD von nicht-typhösen Salmonellen ist, oder ein immunogenes Fragment davon, mit der Fähigkeit, eine Antikörperreaktion gegen nicht-typhöse Salmonellen (NTS) zu erzeugen; oder
(ii) eine/einer Variante/Varianten eines Polypeptids wie in (i) definiert, die die genannte Fähigkeit aufweist, wobei die Impfstoffzusammensetzung ferner ein Adjuvans umfasst.

10. Impfstoffzusammensetzung nach Anspruch 9, wobei das genannte Adjuvans ein immunogenes Polypeptid in der Form eines Fusionsproteins ist, bei dem eine Polypeptidsequenz, wie in (i) oder (ii) definiert, mit einer zweiten Polypeptidsequenz verbunden ist, wobei gegebenenfalls die genannte zweite Polypeptidsequenz ein Adjuvans-Carrierprotein ist.

11. Impfstoffzusammensetzung nach Anspruch 9 oder Anspruch 10, die ein oder mehrere zusätzliche immunogene Polypeptide umfasst.

12. Impfstoffzusammensetzung nach Anspruch 11, wobei die genannten ein oder mehreren zusätzlichen Polypeptide aus Polypeptiden ausgewählt sind, umfassend oder bestehend aus:
(i) ein/einem Außenmembranporin von NTS, ST oder S. paratyphi, oder ein immunogenes Fragment davon; und
(ii) Analoga davon, die die Fähigkeit behalten, eine Antikörperreaktion gegenüber Salmonellen zu erzeugen.

13. Impfstoffzusammensetzung nach Anspruch 9 oder Anspruch 10 für die Verwendung in einem Verfahren für die Behandlung oder Verhinderung einer NTS-Infektion, insbesondere einer STm-Infektion.

14. Impfstoffzusammensetzung nach Anspruch 11 oder Anspruch 12 für die Verwendung in einem Verfahren für die Behandlung oder Verhinderung einer NTS-Infektion, insbesondere einer STm-Infektion und einer Infektion durch S. paratyphi und/oder ST.

## Revendications

1. Polypeptide immunogène qui comprend ou consiste en :
(i) un polypeptide qui est un OmpD de Salmonella non typhoïdique ou un fragment immunogène de celui-ci ayant la capacité à générer une réponse en anticorps contre la Salmonella non typhoïdique (NTS) ; ou
(ii) une variante d'un polypeptide tel que défini au point (i) qui a ladite capacité, pour son utilisation en thérapie.

2. Polypeptide immunogène pour son utilisation selon la revendication 1, dans lequel le polypeptide immunogène est destiné à l'utilisation dans un procédé de traitement ou de prévention d'une infection et/ou une maladie à NTS.

3. Polypeptide immunogène pour son utilisation selon la revendication 2, dans lequel le polypeptide immunogène est destiné à l'utilisation dans un procédé de traitement ou de prévention d'une infection et/ou une maladie à la *Salmonella Typhimurium* (STm).

4. Polypeptide immunogène pour son utilisation selon l'une quelconque des revendications 1 à 3, dans lequel le polypeptide immunogène comprend ou consiste en OmpD pouvant être obtenu à partir de STm ou un fragment immunogène de celui-ci.

5. Polypeptide immunogène pour son utilisation selon la revendication 1, dans lequel le polypeptide immunogène est une protéine de fusion dans laquelle une séquence polypeptidique telle que définie au point (i) ou (ii) est liée à une deuxième séquence polypeptidique.

6. Polypeptide immunogène pour son utilisation selon la revendication 5, dans lequel ladite deuxième séquence polypeptidique est une protéine de support adjuvante.

7. Composition vaccinale comprenant un polypeptide immunogène qui comprend ou consiste en :
(i) un polypeptide qui est un OmpD de Salmonella non typhoïdique ou un fragment immunogène de celui-ci ayant la capacité à générer une réponse en anticorps contre la Salmonella non typhoïdique (NTS) ; ou
(ii) une variante d'un polypeptide tel que défini au point (i) qui a ladite capacité,
la composition vaccinale comprenant en outre un ou plusieurs polypeptides immunogènes supplémentaires.

8. Composition vaccinale selon la revendication 7, dans laquelle ledit/lesdits un ou plusieurs polypeptide(s) immunogène(s) supplémentaire(s) est/sont choisis parmi des polypeptides comprenant ou consistant en :
(i) une porine de membrane externe de NTS, *S. typhi* (ST) ou S. *paratyphi* ou un fragment immunogène de ceux-ci ; et
(ii) des analogues de ceux-ci qui conservent la capacité à générer une réponse en anticorps à la Salmonella.

9. Composition vaccinale comprenant un polypeptide immunogène qui comprend ou consiste en :
(i) un polypeptide qui est un OmpD de Salmonella non typhoïdique ou un fragment immunogène de celui-ci ayant la capacité à générer une réponse en anticorps contre la Salmonella non typhoïdique (NTS) ; ou
(ii) une variante d'un polypeptide tel que défini au point (i) qui a ladite capacité,
dans laquelle la composition vaccinale comprend en outre un adjuvant.

10. Composition vaccinale selon la revendication 9, dans laquelle ledit adjuvant est un polypeptide immunogène sous la forme d'une protéine de fusion dans laquelle une séquence polypeptidique telle que définie au point (i) ou (ii) est liée à une deuxième séquence polypeptidique, éventuellement dans laquelle ladite deuxième séquence polypeptidique est une protéine de support adjuvante.

11. Composition vaccinale selon la revendication 9 ou 10, qui comprend un ou plusieurs polypeptides immunogènes supplémentaires.

12. Composition vaccinale selon la revendication 11, dans laquelle ledit/lesdits un ou plusieurs polypeptide(s) supplémentaire(s) est/sont choisis parmi les polypeptides comprenant ou consistant en :
(i) une porine de membrane externe de NTS, ST ou S. *paratyphi* ou un fragment immunogène de ceux-ci ; et
(ii) des analogues de ceux-ci qui conservent la capacité à générer une réponse en anticorps à la Salmonella.

13. Composition vaccinale selon la revendication 9 ou la revendication 10, pour son utilisation dans un procédé de traitement ou de prévention d'une infection à NTS, en particulier d'une infection à STm.

14. Composition vaccinale selon la revendication 11 ou la revendication 12, pour son utilisation dans un procédé de traitement ou de prévention d'une infection à NTS, en particulier d'une infection à STm, et d'une infection par *S. paratyphi* et/ou ST.
